# EUROPEAN PATENT SPECIFICATION

(11) **EP 4 490 220 B1**
(45) Date of publication and mention of the grant of the patent: **25.02.2026**
(21) Application number: 23711811.2
(22) Date of filing: 09.03.2023
(51) Int. Cl.: C08G 73/02, C08G 75/14, A61K 47/34, C08L 79/02

(54) **AZANAPHTHALENE-FUNCTIONALIZED POLYMERS FOR GENE AND DRUG DELIVERY**
AZANAPHTHALENFUNKTIONALISIERTE POLYMERE ZUR GEN- UND WIRKSTOFFFREISETZUNG
POLYMÈRES FONCTIONNALISÉS PAR AZANAPHTALÈNE POUR L'ADMINISTRATION DE GÈNES ET DE MÉDICAMENTS

(30) Priority: 09.03.2022 NL 2031208
(43) Date of publication of application: 15.01.2025
(73) Proprietor: 20MED Therapeutics B.V., 2333 BD Leiden (NL)
(72) Inventor: ENGBERSEN, Johannes Franciscus Joseph, 2333 BD Leiden (NL); ROELOFS-HAARHUIS, Hendrika Maria, 2333 BD Leiden (NL); VAN DER WAL, Steffen, 2333 BD Leiden (NL); RIP, Jacob, 2333 BD Leiden (NL)
(74) Representative: V.O.
(86) International application number: PCT/NL2023/050112
(87) International publication number: WO 2023/172135

(56) References cited:
- WO-A1-2021/069920
- ADERIBIGBE B A ET AL: "Macromolecular Conjugates of 4- and 8-Aminoquinoline Compounds", JOURNAL OF INORGANIC AND ORGANOMETALLIC POLYMERS, KLUWER ACADEMIC PUBLISHERS-PLENUM PUBLISHERS, NE, vol. 22, no. 2, 1 November 2011 (2011-11-01), pages 429 - 438, XP035026716, ISSN: 1574-1451, DOI: 10.1007/S10904-011-9616-1

## Description

The invention in the field of polymeric nanoparticles for the delivery of biologically active components such as polynucleotides, oligonucleotides, peptides and/or proteins to cells. In particular, the invention is directed to polyamine-containing polymers for these nanoparticles.

Polymeric nanoparticles, and in particular polycationic nanoparticles are used in the medical field to deliver biologically active components such as polynucleotides, oligonucleotides, peptides and/or proteins to cells. See for instance Pudlarz and Szemraj (Open Life Sci. 2018: 13: 285-298). These polymeric nanoparticles comprise polymers, for example polyamine-containing polymers that under physiological conditions can be positively charged in which case the resulting nanoparticles may be referred to as polycationic or simply cationic nanoparticles.

The process of transport, delivery and activation of a payload by nanoparticles is referred to as transfection. In case the payload comprises a nucleic acid, transfection may be used to indicate the process of deliberately introducing nucleic acids into eukaryotic cells. The success of a medical method with the nanoparticles *i.a.* depends on the transfection efficiency. See *i.a.* Kim and Eberwine, Anal. Bioanal. Chem. 2010 Agu:397(8):3173-8 in this respect. The transfection efficiency depends on various factors, including particle and payload stabilization, endocytosis, intracellular transport, localization, and payload unpacking.

Various specific polyamine-containing polymers are known from WO 2012/165953 and references cited therein as well as US 2012/0149630 and WO 2021/069920. These polymers are known for their capacity to deliver siRNA, DNA etc.

Other types of polymers for DNA delivery are known from Piotrowski-Daspit et al. Advanced Drug Delivery Reviews 156 (2020) 119-132 and Van Bruggen et al., PNAS, 117 (2020) (52), 32919-32928.

Quinoline-functionalized polymers are known from *i.a.* Aderibigbe et al. (J. Inorg. Organomet. Polym. (2012), 22:429-438).

Polyamine-containing polymers, such as those specifically described in the aforementioned WO 2012/165953 are known for their good transfection efficiency. However, there remains a desire to further improve the transfection efficiency of such polymers.

The present inventors surprisingly found that transfection efficiency of polymeric nanoparticles based on polyamine-containing polymers can be improved by functionalizing a part of these polyamine-containing polymers with one or more azanaphthalene moieties.

Accordingly, in one aspect, the present invention is directed to a nanoparticle comprising an azanaphthalene-functionalized polymer, wherein said polymer comprises a polyamine segment of formula (I),

-[X-B]ₙ-[X-Q]ₘ- (I)

wherein X is based on a bis(α,β-unsaturated carbonyl) monomer, B is based on an amine monomer, Q is based on an azanaphthalene-containing amine monomer comprising an azanaphthalene moiety, and wherein *m* is 1 or more, and *n* is 1 or more.

It was found that polymers with an increasing content of the azanaphthalene moiety (*i.e.* with an increasing number *m*) lead to a concomitant increase in transfection efficiency in cells. It was however also found that this effect plateaus. Considering that it was also found that polymers based on amine monomers that do not contain an azanaphthalene moiety, in addition to the azanaphthalene-containing amine monomers (*i.e.* polymers wherein *n* is at least 1) may be more easily synthesized in an aqueous solvent, which is preferred, than polymers that are fully based on azanaphthalene-containing amine monomers (*i.e.* polymer wherein *n* is 0). Polymers wherein *n* is 0, generally resulted in poorly water-soluble polymers in slightly acidic to neutral pH ranges. Accordingly, the synthesis of these polymers is considered impractical. The polymers wherein *n* is 0, may be obtained in water-free solvent systems, such as dimethylsulfoxide. However, this typically provides polymers that could not be readily purified using commonly utilized aqueous purification methods.

The incorporation of an amine monomer (*i.e.* the monomer onto which B is based) to obtain the polymer in accordance with the present invention, *i.e. n* is at least 1, resulted in an improved synthesis. Generally, water-soluble polymers are provided at a wide range of azanaphthalene-containing amine monomer (Q) to amine monomer (B) ratios. The polymers may accordingly be synthesized in the preferred aqueous solvents and purified using commonly utilized purification methods.

The azanaphthalene moiety herein means a naphthalene moiety (having the molecular formula C₁₀H₇₋ₓ, wherein x is the number of optional substituents) of which at least one carbon is replaced by a nitrogen atom - *e.g*. mono-azanaphthalene having the molecular formula C₉NH₆₋ₓ, a diazanaphthalene having the molecular formula C₈N₂H₅₋ₓ, etcetera, wherein x is the number of optional substituents. The azanaphthalene moiety may be substituted or unsubstituted. Preferably it is substituted with an alkoxy or a halide substituent.

Particularly suitable and preferred mono-azanaphthalenes for the present invention include benzopyridines including quinolines (also referred to as 1-benzazines or 2,3-benzopyridines) and isoquinolines (also referred to as 2-benzazines or 3,4 benzopyridine). Quinoline moieties gave particular good results and are most preferred. In case the azanaphthalene moiety is a quinoline moiety, which is preferred, the polymer of the invention can be referred to as a quinoline-functionalized polymer.

Particularly suitable diazanaphthalenes for the present invention include 1,2-diazanaphthalenes (also referred to as cinnolines), 1,3-diazanaphthalenes (also referred to as quinazolines), 1,4-diazanaphthalenes (also referred to as quinoxalines), 2,3-diazanaphthalenes (also referred to as phthalazines), 1,8-diazanaphthalenes, 1,7-diazanaphthalenes, 2,7-diazanaphthalenes, 1,6-diazanaphthalenes, 1,5-diazanaphthalenes, and 2,6-diazanaphthalenes.

The term "segment" as used herein is to be understood as a polymeric structure of any length. The polyamine segment according to formula (I) is based on bis(α,β-unsaturated carbonyl) monomer (X) and amine monomers (Q and B), a combination of which result in a repeating unit. In each repeating unit may thus be present either a Q amine (*i.e.* [X-Q]) or a B amine (*i.e.* [X-B]), which can randomly be positioned in the polyamine segment. The amine monomer B does not comprise an azanaphthalene moiety and differs in at least this aspect from the azanaphthalene-containing amine monomer Q. The present polyamine segment can thus be regarded as a copolymer, in particular a random copolymer.

The labels *n* and *m* represent the number of each repeating unit in the polyamine. In the polyamine, Q is always present, thus *m* equal 1 or more, and B is always present: *n* equals 1 or more. The ratio of *n* and *m* may be determined by proton nuclear magnetic resonance spectroscopy (¹H-NMR), wherein the integration of the aromatic signals of Q may be compared with the total polymeric CH₂ integral at a defined spectral window of 1.0 to 2.0 ppm. From this ratio the *m*/*n* ratio may be derived.

In typical embodiments, the sum of *n* and *m* is in the range of 2-250, preferably 5 to 100. The polyamine has a typical weight average molecular weight in the range of 1 to 100 kD, preferably in the range of 5 to 40 kD. The weight average molecular weight may be determined by a gel permeation chromatography (GPC) based method. In particular, the weight average molecular weight may be determined using three G1316B thermostatted (35 °C) columns serially connected. (NOVEMA MAX Medium combination with a molar mass range: 100 - 1 000 000 Da ; PSS NOVEMA Max 30 Å, 5 µm, 8 x 300 mm + 2 x NOVEMA Max 1000 Å 5 µm column 8 x 300 mm, PSS Polymer Standards Service GmbH) with 100 mM NaCl + 0.3% Formic acid as eluent at a flow rate of 1 mL/min and equipped with a refractive index detector. Molecular weights may be determined relative to dextran standards.

### X and B groups

The polyamine may be based on Q in combination with two or more different amines monomer B, for example three different types of amine monomer B. Further, Q may represent a mixture of different azanaphthalene-containing amine monomers. The same applies to the X component, derived from the structure of bis(α,β-unsaturated carbonyl) monomer. X, B and Q in formula (I) may thus each represent a mixture of bis(α,β-unsaturated carbonyl) monomers, general amine monomers and azanaphthalene-containing amine monomers respectively. It may be appreciated that B and Q are typically based on primary and secondary amines, as such amines are generally sufficiently reactive in the polymerization reactions leading to the polymer (*i.e.* the aza-Michael-addition type reactions, *vide infra*)*.*

The polyamine segment according to formula (I), and in particular X, Q and B thereof are defined by the monomers on which they are based, as is common in the art for the sake of clarity and conciseness. It may be appreciated that in a preparation of the polyamine, the X monomers react with the Q and B monomers respectively, due to which their chemical structure alters. For example, during preparation of the polyamine, the monomers on which Q and B are based typically react in an (aza-)Michael-addition type of reaction with the bis(α,β-unsaturated carbonyl) monomer X, after which the double bonds in the α,β-unsaturated carbonyl moieties change into single bonds.

The monomer X is preferably a bis(α,β-unsaturated carbonyl) monomer according to formula (Iₓ), 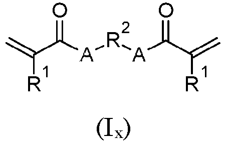 wherein
A is selected from the group consisting of C(R¹)₂, O, N(R¹), S, and combinations thereof,
R¹ is selected from the group consisting of H, halides and C₁-C₂₂ hydrocarbyls such as alkyls, alkenyls and polyalkenyls, which are optionally substituted with one or more halides, and combinations thereof; and
R² is selected from the group consisting of C₁-C₄₀ linear, cyclic and branched hydrocarbylenes, which are optionally substituted and/or interrupted with one or more heteroatoms, and combinations thereof.

Preferably, A in the polyamine segment comprises an N(R₁) in which case the polymer segment can be referred to as a poly(aminoamide). In other words, the polyamine segment is preferably a poly(aminoamide) (PAA) segment.

Hydrocarbyl and hydrocarbylenes as used for the present invention include univalent and bivalent groups formed by removing one or two hydrogen atoms from a hydrocarbon, *e.g*. ethyl, phenyl and ethylene, phenylene, respectively. When interrupted with one or more heteroatoms, these groups can be heteroaliphatic and/or heteroaromatic, as well as linear, branched and/or cyclic.

The monomer B is based on a primary amine according to formula (I_{B1}), 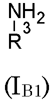 or on a diamine according to formula (I_{B2}), 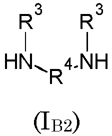 wherein
R³ is independently selected from the group consisting of H, C₁-C₄₀ linear, cyclic and branched hydrocarbyls, which are optionally substituted and/or interrupted with one or more heteroatoms, and combinations thereof; and
R⁴ is selected from the group consisting of C₁-C₄₀ linear, cyclic and branched hydrocarbylenes, which are optionally substituted and/or interrupted with one or more heteroatoms, and combinations thereof.

Preferably, R³ has an overall neutral or negative charge at slightly acidic to neutral pH. With slightly acidic to neutral pH is herein meant a pH between about 6 to about7.

In particular embodiments of the present invention, X is degradable under physiological conditions. Accordingly, X preferably contains a moiety of which one or more bonds readily cleave (*e.g*. due to hydrolysis, reduction, oxidation etc.) under physiological conditions. Examples of such moieties include esters, thioester and disulfide moieties. Disulfide moieties are particularly preferred in this respect. Accordingly, X preferably comprises a disulfide moiety, more preferably R² in formula (Iₓ) comprises a disulfide moiety, even more preferably R² is selected from the group consisting of C₁-C₁₀ hydrocarbylenes, preferably linear alkylenes, interrupted with at least one disulfide moiety, and combinations thereof.

In particular embodiments of the present invention, B comprises a hydroxyl moiety, preferably a primary hydroxyl moiety. Accordingly, at least one or more of R³ in formulae (I_{B1}) and/or (I_{B2}) comprises a hydroxyl moiety, more preferably at least one or more of R³ in formulae (I_{B1}) and/or (I_{B2}) is selected from the group consisting of C₁-C₁₀ hydroxyhydrocarbyl, most preferably linear alkyl C₁-C₁₀ hydroxide, and combinations thereof.

Particularly suitable monomers on which B may be based, have biological activity and/or confer water solubility. Particularly suitable monomers include 4-aminobutanol (ABOL) and 5-aminopentanol (APOL) and other hydroxyl-comprising monomers. Preferably, the monomer is neutral (*i.e.* not charged) under physiological conditions (*i.e.* at pH 7 at 20 °C) when incorporated into the polymer. Incorporation of these neutral monomers like ABOL and APOL resulted in an better transfection efficiency compared to monomers that are positively charged under physiological conditions when incorporated into the polymer (see *e.g.* Lin et al. Bioconjugate Chem. 2007, 18, 1, 138-145). Examples of such monomers that are positively charged include amine-monomers including an additional amine group (*e.g.* an aliphatic tertiary amine) like dimethylaminopropylamine and N-(3-aminopropyl)-morpholine). Monomers that are positively charged under physiological conditions when incorporated into the polymer are thus less preferred for B.

### Q and the azanaphthalene moiety

The Q in the polyamine comprises the azanaphthalene moiety. The azanaphthalene moiety may optionally be substituted, as further elucidated herein.

In preferred embodiment, Q is based on a primary amine according to formula (I_{Q1}), 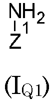 and/or on a diamine according to formula (I_{Q2}), 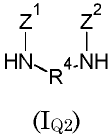 wherein
Z¹ represents a group comprising the azanaphthalene moiety;
Z² represents a group comprising the azanaphthalene moiety or is selected from the group consisting of H, C₁-C₄₀ linear, cyclic and branched hydrocarbyls, which are optionally substituted and/or interrupted with one or more heteroatoms, and combinations thereof; and
R⁴ can be as defined herein-above for formula (I_{B2}).

In formula (I_{Q2}), at least one azanaphthalene moiety is present. In certain embodiments, Q according to formula (I_{Q2}) comprises only an azanaphthalene moiety in Z¹ and none in Z². In other embodiments, Q according to formula (I_{Q2}) comprises azanaphthalene moieties in both Z¹ and in Z². The polyamine according to the present invention may also comprise a mixture of various Q groups, each group having a different structure and/or comprising a different amount of azanaphthalene moieties.
In particular embodiments, Z¹ and optionally Z² independently represent a moiety according to any of the following structures, 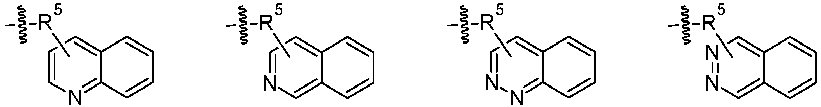 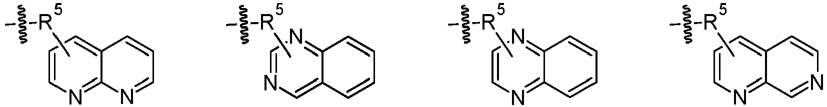 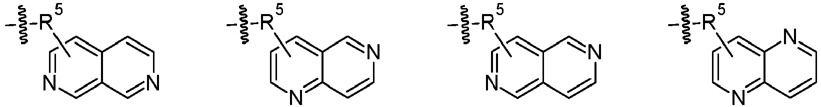 wherein R⁵ is selected from the group consisting of C₁-C₄₀, preferably C₁-C₁₀, more preferably C₁-C₆ linear, cyclic and branched hydrocarbylenes, which are optionally substituted and/or interrupted with one or more heteroatoms, and combinations thereof; and wherein the azanaphthalene moiety is optionally substituted with one or more substituents, preferably selected from the group consisting of halides, alkyls and alkoxides, optionally substituted with one or more heteroatoms.

The azanaphthalene moiety can be attached to the primary or secondary amine group(s) of formulae (I_{Q1}) and (I_{Q2}) in various ways. Preferably, the moiety is attached via a spacer R⁵ to the backbone of the polyamine segment.

More preferably, Z¹ and optionally Z² in any of formulae (I_{Q1}) and (I_{Q2}) independently represent a quinoline moiety of formula (I_{Z1}), (I_{Z2}) or (I_{Z3}), preferably a quinoline moiety of formula (I_{Z3}),

| | | |
|---|---|---|
| 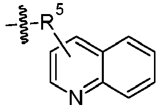 | 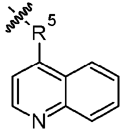 | 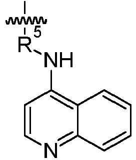 |
| (I_{Z1}) | (I_{Z2}) | (I_{Z3}) |

wherein R⁵ is selected from the group consisting of C₁-C₄₀, preferably C₁-C₁₀, more preferably C₁-C₆ linear, cyclic and branched hydrocarbylenes, which formulae are optionally substituted and/or interrupted with one or more heteroatoms, and combinations thereof, of which a preferred example is given in I_{Z3}.

The azanaphthalene moiety may optionally be substituted with one or more substituents, preferably selected from the group consisting of halides, alkyls and alkoxides, both which groups may again be optionally substituted with one or more heteroatoms. In a particularly preferred embodiment, the quinoline moiety comprises a substituent on the 7-position and Z¹ and optionally Z² in any of formulae (I_{Q1}) and (I_{Q2}) independently represent a moiety of formula (I_{Z4}) 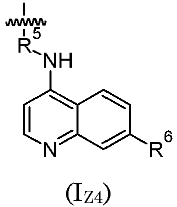 wherein R⁶ is selected from the group consisting of halides, alkyls, alkoxides and mixtures thereof, optionally substituted with one or more heteroatoms, preferably halides, more preferably wherein R⁶ is Cl.

The present inventors found that the spacer R⁵ can be of a relatively simple structure, for example a linear unsubstituted alkylene such as butylene (C₄H₈), hexylene (C₆H₁₂) or octylene (C₈H₁₆) while retaining the advantageous effect of improving transfection. Thus, although it is possible to use a spacer that comprises a quinuclidine such as present in the natural product quinine, this is not required.

### General polymer formula and a core

In embodiments wherein X, B and Q are based on monomers according to formulae (I_{X}), (I_{B1}) or (I_{B2}), and (I_{Q1}) or (I_{Q2}), the polyamine can be represented by any of formulae (IIa)-(IId) and combinations thereof, wherein *n, m, p,* A, R¹, R², R³, R⁴, Z¹ and Z² can be as defined for (I), (I_{X}), (I_{B1}), (I_{B2}), (I_{Q1}) and (I_{Q2}).

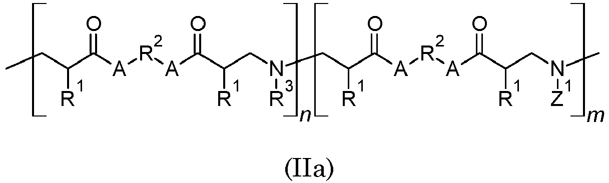

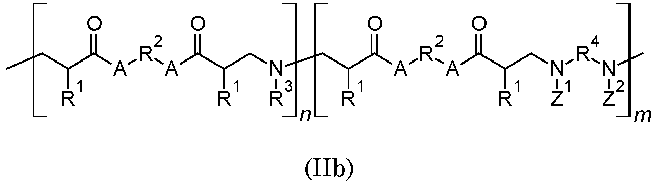

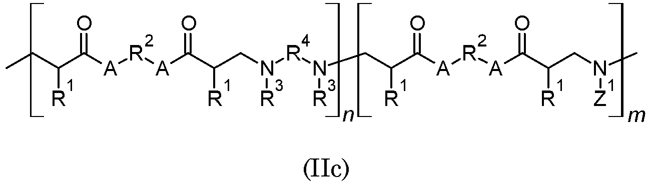

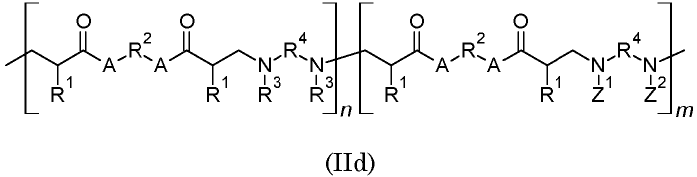

In particular embodiments of the present invention, the polyamine is linked to a core, preferably a polymeric core (T). Accordingly, in particular embodiments of the present invention, the polyamine segment and preferably the polymer according to the present invention has a structure according to formula (III), wherein *n* and *m* can be as defined for formula (I) and *q* represents the number of poly(amino amide) segments per core (T), *i.e.* the number of arms. In a typical embodiment, *q* is in the range of 1 to 64.

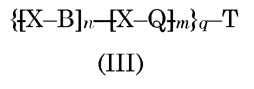

In a particular preferred embodiment, the polyamine segment and preferably the polymer according to the present invention has a structure according to any of formulae (IVa), (IVb), (IVc), (IVd), and combinations thereof, wherein *n, m,* A, R¹, R², R³, R⁴, Z¹ and Z² can be as defined for formulae (I), (I_{X}), (I_{B1}), (I_{B2}), (I_{Q1}) and (I_{Q2}). 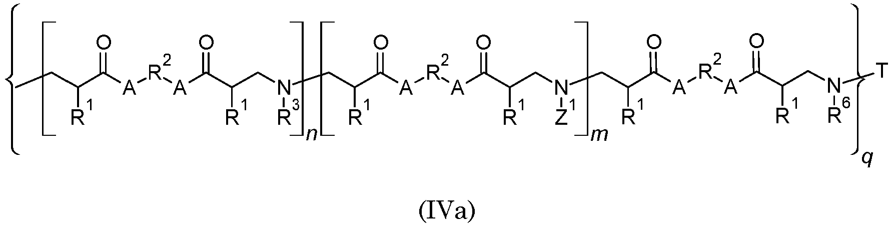 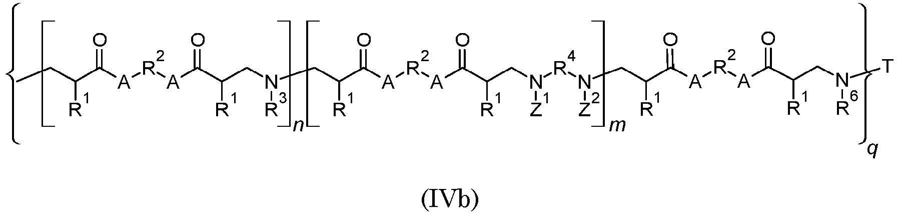 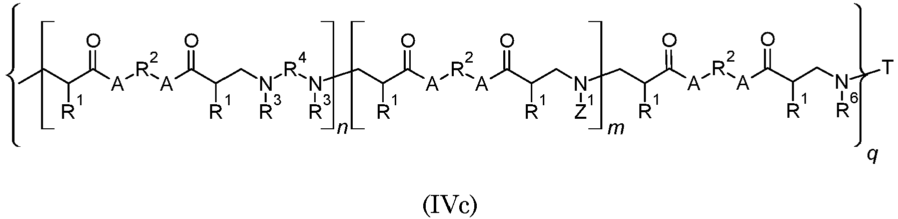 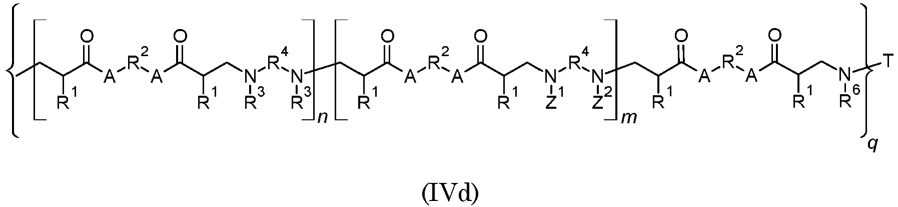 Further, in formulae (III) and (IVa)-(IVd), T represents a core that preferably has an weight average molecular weight M_{w} of about 60 to about 25000; *q* is in the range of 1 to 64; and R⁶ is selected from the group consisting of H, C₁-C₄₀ linear, cyclic and branched hydrocarbyls, which are optionally substituted and/or interrupted with one or more heteroatoms, and combinations thereof.

The core T is generally based on a structure with one or more primary and/or secondary amines. These amines are represented in formulae (IVa)-(IVd) with the group NR⁶. Particularly suitable cores include oligo- or polyamine cores such as 1,2-ethylenediamine, tris-(2-aminoethyl)amine as well as polymeric cores bearing primary and/or secondary amines such as polyethyleneimines (PEI), poly(aminoamide) polymers or dendrimers (PAMAM), polypropylene imines (PPI), poly(ester amine) polymers (PEAN) and poly(ether amine) polymers (PEAC) and other polymers as described in WO 2012/165953 (referred therein a POL). PEI is particularly preferred as the core and most preferred is PEI 800 with on average 6 primary amines in this respect, as this gave particular good results. It may be appreciated that both ends of the poly(aminoamide) polymer can potentially react with POL, which may result in two or more cores being incorporated into the same macromolecule. In certain embodiments however, reaction conditions can be chosen to limit crosslinking.

The present inventors found that the relative amount of the azanaphthalene-containing amine monomer Q vis-à-vis the amine monomer B in the polyamine and/or the polymer influences the transfection efficiency and toxicity. Additionally, it was found that the higher the relative amount of Q becomes, the more the solubility concomitantly decreases during the synthesis of the polymer and similarly results in polymers with lower solubility in aqueous systems.

The relative amount of Q vis-à-vis B can be expressed as the ratio *m* to *n.* As the amine monomer B is present, *n* is at least 1 and the ratio may accordingly be 1:1, *i.e.* 1. However, preferably the polyamine segment and/or the polymer contains more B than Q. In particular embodiments, the ratio *m* to *n* is in the range of 1:20 or more (*i.e.* 0.05 or more, *e.g.* 0.05 to 20). Preferably, the ratio *m* to *n* is in the range of 1:10 to 10:1, more preferably in the range of 1:5 to 2:1, most preferably in the range of 1:4 to 1:1 such as about 1:3, as particular good results were obtained in that range.

Suitable embodiments of the monomers according to formulae (Iₓ) and (I_{B1}) and (I_{B2}) for the polymer according to the present invention are described in WO 2012/165953.
Accordingly, preferably, the following applies to the polyamine segment and/or the polymer according to the present invention and/or its monomers on which it is based:
- R² is independently selected from the group consisting of:
   a) C₁ - C₄₀ alkylene, wherein the alkylene group may be linear or branched and is optionally substituted and/or is optionally (partly) unsaturated and/or is optionally interrupted by one or more heteroatoms, wherein the heteroatoms are independently selected from O, N and S, and/or wherein the alkylene group is interrupted by one or more -S-S- groups;
   b) C₃ - C₄₀ cycloalkylene, wherein the cycloalkylene group is optionally substituted and/or is optionally (partly) unsaturated and/or optionally comprises one or more heteroatoms in the ring, wherein the heteroatoms are independently selected from O, N and S, and/or wherein the cycloalkylene group is interrupted by one or more -S-S-groups outside the ring;
   c) C₆ - C₄₀ arylene, wherein the arylene group is optionally substituted;
   d) C₆ - C₄₀ heteroarylene, wherein the heteroarylene group comprises one or more heteroatoms independently selected from O, N and S and/or wherein the heteroarylene group is optionally substituted;
   e) C₇ - C₄₀ alkylarylene wherein the alkylarylene group is optionally substituted and/or wherein an alkyl part of the alkylarylene group is linear or branched and is optionally (partly) unsaturated and/or is optionally interrupted by one or more heteroatoms, wherein the heteroatoms are independently selected from 0, N and S, and/or wherein an alkyl part of the alkylarylene group is interrupted by one or more -S-S- groups;
   f) C₇ - C₄₀ alkylheteroarylene, wherein the alkylheteroarylene group comprises one or more heteroatoms independently selected from O, N and S and/or wherein the alkylheteroarylene group is optionally substituted, and/or wherein an alkyl part of the alkylheteroarylene group is linear or branched and is optionally (partly) unsaturated and/or is optionally interrupted by one or more heteroatoms, wherein the heteroatoms are independently selected from 0, N and S, and/or wherein an alkyl part of the alkylheteroarylene group is interrupted by one or more -S-S- groups; and
   g) a group wherein two C₇ - C₄₀ (hetero)arylene groups and/or C₇ - C₄₀ alkyl(hetero)arylene groups are connected to each other by a -S-S-group, wherein the alkyl part of the alkyl(hetero)arylene group is linear or branched and is optionally (partly) unsaturated and/or is optionally interrupted by one or more heteroatoms, wherein the heteroatoms are independently selected from O, N and S;
   and combinations thereof;
- R³ is independently selected from the group consisting of:
   a) H;
   b) C₁ - C₁₀ alkyl, wherein the alkyl group may be linear or branched and is optionally substituted and/or is optionally (partly) unsaturated and/or is optionally interrupted by one or more heteroatoms, wherein the heteroatoms are independently selected from O, N and S;
   c) C₃ - C₁₂ cycloalkyl, wherein the cycloalkyl group is optionally substituted and/or is optionally (partly) unsaturated and/or optionally comprises one or more heteroatoms in the ring, wherein the heteroatoms are independently selected from O, N and S;
   d) C₆ - C₁₂ aryl, wherein the aryl group is optionally substituted;
   e) C₆ - C₁₂ heteroaryl, wherein the heteroaryl group comprises one or more heteroatoms, wherein the heteroatoms are independently selected from O, N and S, and wherein the heteroaryl group is optionally substituted;
   f) C₇ - C₁₄ alkylaryl wherein the alkylaryl group is optionally substituted and/or wherein an alkyl part of the alkylarylene group is linear or branched and is optionally (partly) unsaturated and/or is optionally interrupted by one or more heteroatoms, wherein the heteroatoms are independently selected from O, N and S; and
   g) C₇ - C₁₄ alkylheteroaryl, wherein the alkylheteroaryl group comprises one or more heteroatoms independently selected from O, N and S and/or wherein the alkylheteroarylene group is optionally substituted, and/or wherein an alkyl part of the alkylheteroarylene group is linear or branched and is optionally (partly) unsaturated and/or is optionally interrupted by one or more heteroatoms, wherein the heteroatoms are independently selected from O, N and S;
   and combinations thereof, preferably wherein R³ has an overall neutral or negative charge at slightly acidic to neutral pH;
- R⁴ is independently selected from the group consisting of
   a) C₁ - C₁₂ alkylene, wherein the alkylene group may be linear or branched and is optionally substituted and/or is optionally (partly) unsaturated and/or is optionally interrupted by one or more heteroatoms, wherein the heteroatoms are independently selected from O, N and S;
   b) C₃ - C₁₂ cycloalkylene, wherein the cycloalkylene group is optionally substituted and/or is optionally (partly) unsaturated and/or optionally comprises one or more heteroatoms in the ring, wherein the heteroatoms are independently selected from O, N and S;
   c) C₆ - C₁₂ arylene, wherein the arylene group is optionally substituted;
   d) C₆ - C₁₂ heteroarylene, wherein the heteroarylene group comprises one or more heteroatoms independently selected from O, N and S and/or wherein the heteroarylene group is optionally substituted;
   e) C₇ - C₁₂ alkylarylene wherein the alkylarylene group is optionally substituted and/or wherein an alkyl part of the alkylarylene group is linear or branched and is optionally (partly) unsaturated and/or is optionally interrupted by one or more heteroatoms, wherein the heteroatoms are independently selected from O, N and S; and
   f) C₇ - C₁₂ alkylheteroarylene, wherein the alkylheteroarylene group comprises one or more heteroatoms independently selected from O, N and S and/or wherein the alkylheteroarylene group is optionally substituted, and/or wherein an alkyl part of the alkylheteroarylene group is linear or branched and is optionally (partly) unsaturated and/or is optionally interrupted by one or more heteroatoms, wherein the heteroatoms are independently selected from O, N and S;
   and combinations thereof;
- R⁶ is independently selected from the group consisting of
   a) H;
   b) C₁ - C₁₀ alkyl, wherein the alkyl group may be linear or branched and is optionally substituted and/or is optionally (partly) unsaturated and/or is optionally interrupted by one or more heteroatoms, wherein the heteroatoms are independently selected from O, N and S;
   c) C₃ - C₁₂ cycloalkyl, wherein the cycloalkyl group is optionally substituted and/or is optionally (partly) unsaturated and/or optionally comprises one or more heteroatoms in the ring, wherein the heteroatoms are independently selected from O, N and S;
   d) C₆ - C₁₂ aryl, wherein the aryl group is optionally substituted;
   e) C₆ - C₁₂ heteroaryl, wherein the heteroaryl group comprises one or more heteroatoms, wherein the heteroatoms are independently selected from O, N and S, and wherein the heteroaryl group is optionally substituted;
   f) C₇ - C₁₄ alkylaryl wherein the alkylaryl group is optionally substituted and/or wherein an alkyl part of the alkylarylene group is linear or branched and is optionally (partly) unsaturated and/or is optionally interrupted by one or more heteroatoms, wherein the heteroatoms are independently selected from O, N and S; and
   g) C₇ - C₁₄ alkylheteroaryl, wherein the alkylheteroaryl group comprises one or more heteroatoms independently selected from O, N and S and/or wherein the alkylheteroarylene group is optionally substituted, and/or wherein an alkyl part of the alkylheteroarylene group is linear or branched and is optionally (partly) unsaturated and/or is optionally interrupted by one or more heteroatoms, wherein the heteroatoms are independently selected from O, N and S;
   and combinations thereof.

### Nanoparticles and medical use

The present invention is directed to nanoparticles comprising the azanaphthalene-functionalized polymer that comprises the polyamine segment as described herein.

Under particular conditions, the azanaphthalene-functionalized polymer can self-assemble into nanoparticles. In addition to the azanaphthalene-functionalized polymer, these nanoparticles further contain a biologically active component that can be used for medical purposes. In general, the biological active component is embedded within the azanaphthalene-functionalized polymer. The biological active component may be any material or combination of materials that can induce a biological or physical response in the human body. Examples for the biologically active component include nucleotides, polynucleotides, oligonucleotides, peptides, proteins and combinations thereof. These for instance include all types of RNA and DNA and their derivatives and analogues, *i.a.* plasmid DNA, dbDNA, hpDNA, c3DNA, minicircles, phosphorodiamidate morpholino oligomers (PMOs), siRNA, mRNA, miRNA, endless RNA, circular RNA, single and/or double stranded RNA including designed guide RNAs (gRNA or sgRNA) used in gene editing techniques. The origin of these materials is irrelevant, thus any artificially constructed or chemically modified nucleotide, oligonucleotides, polynucleotides, oligopeptides, polypeptides and/or proteins are also to be understood to be included by the term biologically active payload. One or more gene constructs are therefore also to be considered a biologically active payload. Moreover, the polynucleotide, oligonucleotide, peptide, nucleotide and/or protein may be based on natural-occurring building blocks, but also on non-natural occurring building blocks such as non-natural nucleosides or non-natural amino acids. Although the present nanoparticles are particularly suitable for polymeric and oligomeric biologically active components, which are accordingly preferred, the biologically active component may also be non-polymeric and non-oligomeric and may include any active pharmaceutical ingredient.

The nanoparticles in accordance with the present invention may further comprise a non-functionalized polymer. With non-functionalized polymer is herein meant a polymer that is not functionalized with a azanaphthalene moiety. The structure of the non-functionalized polymer may be similar to the azanaphthalene-functionalized polymer, with the main difference being the absence of the azanaphthalene moiety. Suitable polymers in this respect are described in WO 2012/165953.

For example, in preferred embodiments, the non-functionalized polymer comprises non-functionalized polyamine segment according to formula (V), more preferably according to formula (VIa), (VIb) or a combination thereof.

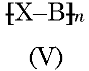

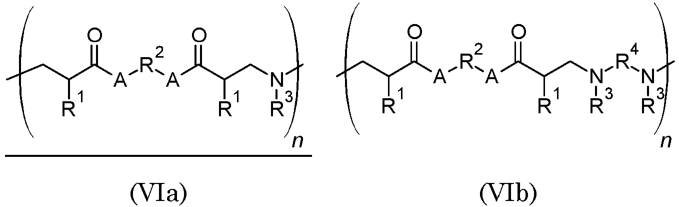

Moreover, similar to the azanaphthalene-comprising polymer, the non-functionalized polymer for use in the nanoparticles according to the present invention may comprise a core (T), and may accordingly have a structure according to formula (VII), more preferably according to formula (IIXa), (IIXb) or a combination thereof.

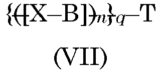

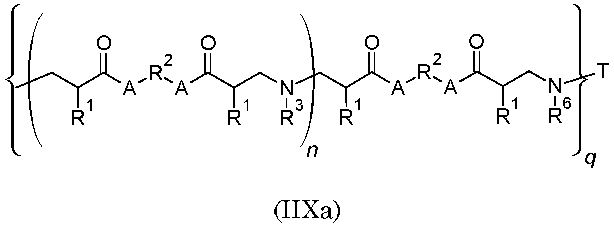

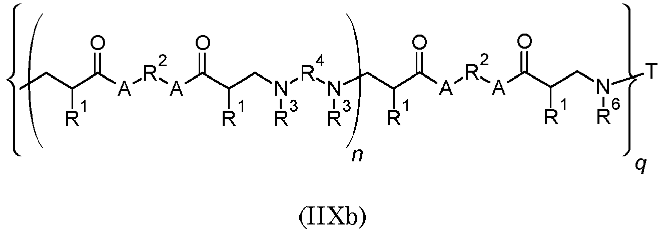

In formula (V), (VIa), (VIb), (VII), (IIXa) and (IIXb), the variables X, B, *n,* A, R₁, R₂, R₃, R₄, R₆, T and *q* may independently be as defined herein for the azanaphthalene-functionalized polymer. With independently is meant that said substituents and groups represented by the variables may be the same or different for the azanaphthalene-functionalized polymer and non-functionalized polymer present in the nanoparticles. In preferred embodiments, both the azanaphthalene-functionalized polymer and non-functionalized polymer present in the nanoparticles have the same substituents and groups represented by X, B, A, R₁, R₂, R₃, R₄, R₆, T and *q*, and preferably wherein *n* of the non-functionalized polymer equals *n* + *m* of the azanaphthalene-functionalized polymer as well.

The non-functionalized polymer is optionally present in the nanoparticles and in typical embodiment, the nanoparticles are only based on the azanaphthalene-functionalized polymer and not on the non-functionalized polymer. This is particularly preferred for good homogeneity of the nanoparticles and their properties. If the non-functionalized polymer is present, the ratio of the non-functionalized polymer and the azanaphthalene-functionalized polymer in the nanoparticles can be suitably varied. Factors that may influence the optimal ratio include the ratio of Q and B in the azanaphthalene-functionalized polymer, the (structural) similarity between the non-functionalized polymer and the azanaphthalene-functionalized polymer and the biologically active ingredient. Preferably, the ratio of the non-functionalized polymer and the azanaphthalene-functionalized polymer in the nanoparticles is such that the molecular ratio of Q to B (counting the sum of B units in the non-functionalized polymer and in the azanaphthalene-functionalized polymer) is in the range of 1:20 or with higher Q content, preferably in the range of 1:10 to 10:1, more preferably in the range of 1:5 to 2:1, most preferably in the range of 1:4 to 1:1 such as about 1:3.

The azanaphthalene-functionalized polymer and the nanoparticles show improved transfection efficiency compared to nanoparticles based on non-functionalized polymers only. The nanoparticles may suitably be used for *in vitro* or *in vivo* transfection with nucleic acids in eukaryotic cells to express proteins, that are advantageous for medical purposes. A further aspect of the present invention is accordingly directed to a method for use of the azanaphthalene-functionalized polymer and the nanoparticles in a medical treatment (medical method). In other words, the present invention is further directed to the azanaphthalene-functionalized polymer and the nanoparticles for use as a medicament.

The term medical treatment herein includes *in vivo* transfection with nucleic acids in eukaryotic cells to express proteins, and includes curative treatments, preventive treatments, prophylactic treatments, diagnostic treatments, and the like.

Particularly, the azanaphthalene-functionalized polymer and/or the nanoparticles can be used as a vaccine, such as a prophylactic vaccine and/or for use as a therapeutic vaccine. The vaccine comprising the nanoparticles may accordingly be used to express therapeutic proteins. The most preferred administration method for the vaccine is typically injection, such as intradermal, subcutaneous, intramuscular injection using parental administration or alternatively using microneedle administration or needle-free injection methods. Other methods, such as oral administration or intranasal administration, are also to be included. Preferably, the polymer-coated nanoparticle is for use as a vaccine by injection, *e.g.* by intramuscular injection.

As used herein, the singular forms "a", "an" and "the" are intended to include the plural forms as well, unless the context clearly indicates otherwise. The term "and/or" includes any and all combinations of one or more of the associated listed items. It will be understood that the terms "comprises" and/or "comprising" specify the presence of stated features but do not preclude the presence or addition of one or more other features.

The present invention can be illustrated with the following nonlimiting examples.

### Example 1 - Synthesis of N1-(7-chloroquinolin-4-yl)-hexane-1,6-diamine (Q6)

1,6-diamino hexane (20.6 g, 0.18 mol) and 4,7-dichloroquinoline (10.3 g, 0.052 mol) were heated to about 150 °C while stirring, thereby melting the solids and the resulting mixture is stirred for 3 hours under nitrogen. To the reaction mixture 50 mL methanol was added and subsequently 50 mL 1M NaOH solution was slowly added while stirring. A visible precipitate was formed. The precipitate was filtered off and the filtrate was further purified by crystallization or silica column chromatography to furnish N1-(7-chloroquinolin-4-yl)-hexane-1,6-diamine (Q6).

### Example 2 - Synthesis of p(CBA-ABOL-Q)/PEI

4-amino-1-butanol (ABOL, 0.60 g, 6.75 mmol), Q6 (0.60 g, 2.16 mmol) and cystamine bis(acrylamide) (CBA, 2.60, 10.0 mmol) were dissolved in methanol (10 mL) at 50 °C under a N₂ atmosphere. MilliQ (2 mL) and CaCl₂ (0.44 g, 4.0 mmol) were added, and the mixture was stirred at 50 °C for 48 hours.

A branched PEI 800 PEI solution in MilliQ water was prepared by weighing 100 mg of PEI in a test tube, adding 1 mL of MilliQ water and mixing to dissolve. Then, 0.680 mL of the prepared PEI solution (100mg/mL) was added to the reaction mixture and allowed to stir at 50°C for 48 hours.

Subsequently the reaction mixture was acidified using 6M HCl to pH 4 and solvents were evaporated to obtain the crude quinoline-functionalized polymer that is further purified by dialysis.

### Example 3 - In vitro transfection efficiency of mRNA-GFP loaded nanoparticles with increasing quantities of Q6 in the used polymers

Polymers p(CBA-ABOL-Q)/PEI were synthesized similarly as in example 2 with 5 mmol CBA and the amounts of ABOL and Q as described in table 1 as feedstock for the polymerization and 220 mg of CaCl₂ as catalyst. 340 µL of PEI stock (100 mg/mL) was added after 48 hours and allowed to stir for 72 hours.

**Table 1 - Amounts of ABOL and Q6 in the different tested polymers**

| | ABOL (mmol) | Q6 (mmol) | Q6 (%) |
|---|---|---|---|
| 1 | 4.16 | 0.34 | 7.5 |
| 2 | 3.83 | 0.68 | 15 |
| 3 | 3.38 | 1.13 | 25 |
| 4 | 2.93 | 1.58 | 35 |
| 5 | 2.25 | 2.25 | 50 |

A solution was made of 120 ng/µL mRNA encoding eGFP (Trilink, cat# L-7201-1000) in 10 mM Histidine pH 6.5. The polymers from table 1 were dissolved at a concentration of 3 mg/mL in 10mM Histidine pH 6.5. The 120 ng/µL solution of mRNA was added 1:1 (v/v) to the cationic polymers. The solutions were incubated for at least 15 min at room temperature before used for transfections.

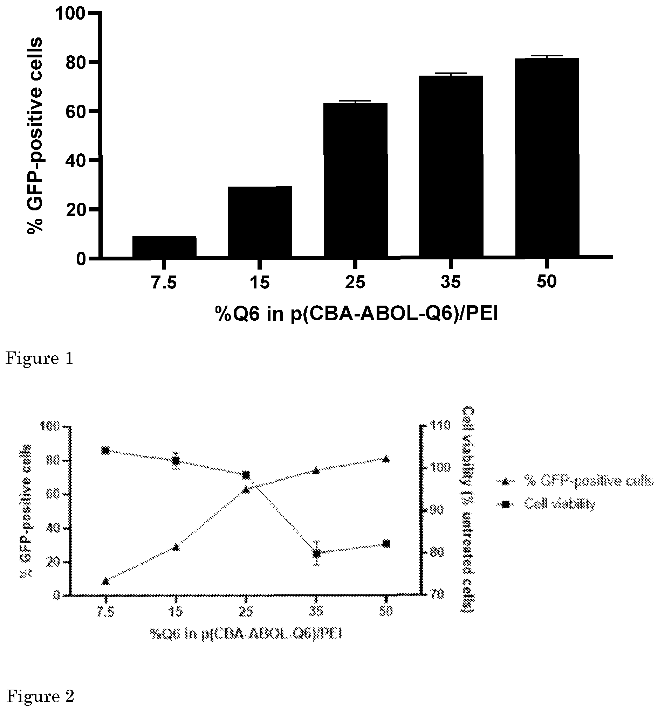

COS7 cells were seeded in 48-wells cell culture plates (1.6x10⁴ cells per well) in culture medium DMEM with 10% FBS. Twenty-four hours after seeding the cells the culture medium was replaced with DMEM with 10% FBS and 2% 1M HEPES pH 7.2 and 15 µg polymer/mL of the in table 1 listed polymers with mRNA. After 24 hours the cells were analyzed for the expression of eGFP using FACS analysis. The percentage cells positive for eGFP increased with increasing amounts of Q6 in the polymers as shown in Figure 1. The viability of the cells was determined with Alamar blue reagent. The viability of the cells decreased with increasing amounts of Q6 in the used polymers. The percentage cells positive for eGFP (identical to the percentages illustrated in Figure 1) and the cell viability are shown in Figure 2.

### Example 4 - Number of transfected cells with mRNA-EGFP loaded nanoparticles with and without Q6

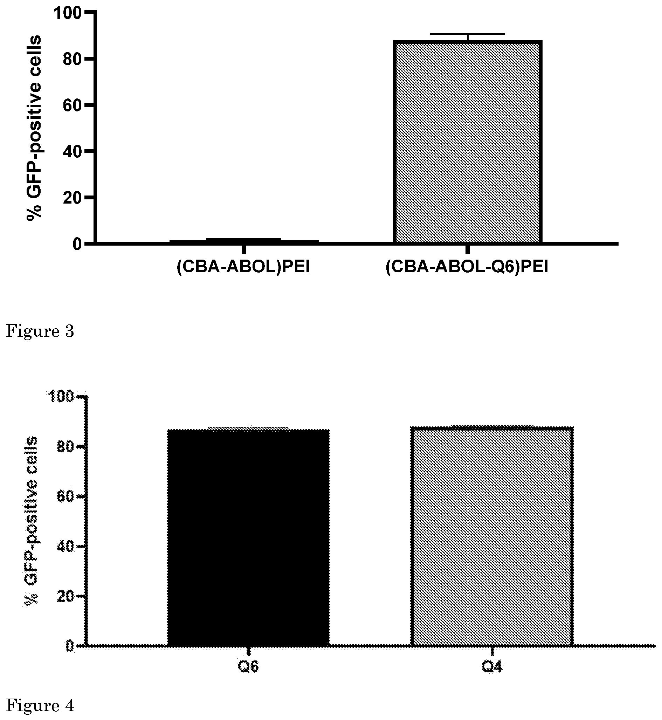

Polymers p(CBA-ABOL-Q6)PEI and p(CBA-ABOL)PEI were synthesized and tested in transfection as described in examples 2 and 3. Polymer p(CBA-ABOL-Q6)PEI containing 25% Q6 was compared to polymer without Q6. C₂C₁₂ cells were transfected with 50 ug polymer /mL culture medium during 24 hours and the number of eGFP positive cells was quantified by FACS analysis. The results are given in Figure 3.

### Example 5 - Transfection efficiency of polymers with Q6 and N1-(7-chloroquinolin-4-yl)-butane-1,4-diamine (Q4)

Q4 was made following a similar procedure as described in Example 1 substituting 1,4-diaminobutane for 1,6-diaminohexane. Q4-containing polymers are synthesized following the same procedure as described in Example 2. The *in vitro* transfection efficiency of mRNA-eGFP loaded nanoparticles based on 25% Q6 and 25% Q4 are determined and compared. COS7 cells were transfected with 30 ug polymer /mL culture medium during 4 hours after which the cell culture medium was replaced. The number of eGFP positive cells was quantified by FACS analysis 24 hours later. The results are given in Figure 4.

### Example 6 - RNA encapsulation stability of nanoparticles with and without Q6

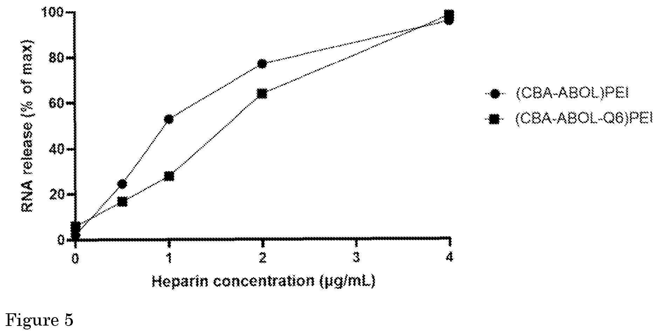

Polymers p(CBA-ABOL-Q6)PEI and p(CBA-ABOL)PEI were synthesized and used in nanoparticle formulations with RNA as described in examples 2 and 3. Polymer p(CBA-ABOL-Q6)PEI containing 25% Q6 was compared to a polymer incorporating only ABOL monomer without Q6. The formed nanoparticles were used in a release assay using heparin competition to displace the RNA nucleic acid from the nanoparticle. Increasing the heparin concentration leads to increasing competition with the binding of RNA to the polymers, which causes release of RNA from the nanoparticles. Released RNA was then quantified using Ribogreen reagent. Higher concentrations of heparin are needed to release RNA from the Q6 containing nanoparticles, indicating stronger interaction between the Q6-containing polymer and RNA and increased nanoparticles stability . The results are given in Figure 5.

## Claims

1. Nanoparticles comprising an azanaphthalene-functionalized polymer and a biologically active component selected from the group consisting of nucleotides, polynucleotides, oligonucleotides, peptides, proteins and combination thereof, wherein said polymer comprises a polyamine segment of formula (I),
-[X-B]ₙ-[X-Q]ₘ- (I)
wherein X is based on a bis(α,β-unsaturated carbonyl) monomer, B is based on an amine monomer, Q is based on an azanaphthalene-containing amine monomer comprising an azanaphthalene moiety, *m* is at least 1, and *n* is at least 1, preferably *n* + *m* is in the range of 2-250.

2. Nanoparticle according to claim 1, wherein the azanaphthalene moiety is selected from the group consisting of benzopyridines including 1-benzazines (quinolines) and 2-benzazines (isoquinolines) and diazanaphthalenes including 1,2-diazanaphthalenes (cinnolines), 1,3-diazanaphthalenes (quinazolines), 1,4-diazanaphthalenes (quinoxalines), 2,3-diazanaphthalenes (phthalazines), 1,8-diazanaphthalenes, 1,7-diazanaphthalenes, 2,7-diazanaphthalenes, 1,6-diazanaphthalenes, 1,5-diazanaphthalenes, and 2,6-diazanaphthalenes, preferably benzopyridines, more preferably quinolines.

3. Nanoparticle according to any of the previous claims, wherein X is based on a bis(α,β-unsaturated carbonyl) monomer according to formula (Iₓ), 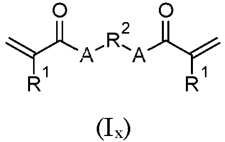 wherein
A is selected from the group consisting of C(R¹)₂, O, N(R¹), S, and combinations thereof,
R¹ is selected from the group consisting of H, halides and C₁-C₂₂ hydrocarbyls such as alkyls, alkenyls and polyalkenyls, which are optionally substituted with one or more halides, and combinations thereof; and
R² is selected from the group consisting of C₁-C₄₀ linear, cyclic and branched hydrocarbylenes, which are optionally substituted and/or interrupted with one or more heteroatoms, and combinations thereof.

4. Nanoparticle according to any of claims 1-2, wherein X comprises a disulfide moiety, preferably wherein X is as defined in claim 3 and wherein R² comprises a disulfide moiety, more preferably wherein R² is selected from the group consisting of C₁-C₁₀ hydrocarbylenes, preferably linear alkylenes, interrupted with at least one disulfide moiety, and combinations thereof.

5. Nanoparticle according to any of the previous claims, wherein B is based on a primary amine according to formula (I_{B1}), 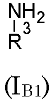
or on a diamine according to formula (I_{B2}), 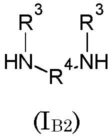
wherein
R³ is independently selected from the group consisting of H, C₁-C₄₀ linear, cyclic and branched hydrocarbyls, which are optionally substituted and/or interrupted with one or more heteroatoms, and combinations thereof; and R⁴ is selected from the group consisting of C₁-C₄₀ linear, cyclic and branched hydrocarbylenes, which are optionally substituted and/or interrupted with one or more heteroatoms, and combinations thereof.

6. Nanoparticle according to any of claims 1-4, wherein B comprises a hydroxyl moiety, preferably wherein B is as defined in claim 5 and wherein R³ comprises a hydroxy moiety, more preferably wherein R³ is selected from the group consisting of C₁-C₁₀ hydrocarbyl hydroxide, preferably linear alkyl C₁-C₁₀ hydroxide, and combinations thereof.

7. Nanoparticle according to any of the previous claims, wherein Q is based on a primary amine according to formula (I_{Q1}), 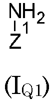
and/or on a diamine according to formula (I_{Q2}), 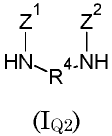
wherein
Z¹ represents a group comprising the azanaphthalene moiety;
Z² represents a group comprising the azanaphthalene moiety or is selected from the group consisting of H, C₁-C₄₀ linear, cyclic and branched hydrocarbyls, which are optionally substituted and/or interrupted with one or more heteroatoms, and combinations thereof;
R⁴ is as defined in claim 5.

8. Nanoparticle according to the previous claim, wherein Z¹ and optionally Z² independently represent a moiety according to any of the following structures, 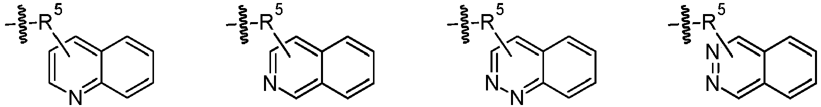 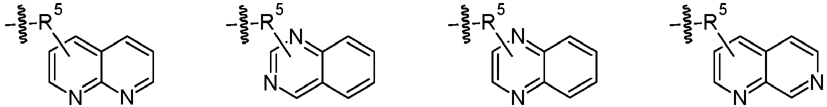 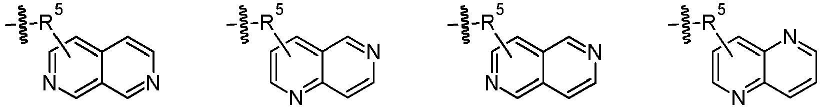
wherein R⁵ is selected from the group consisting of C₁-C₄₀, preferably C₁-C₁₀, more preferably C₁-C₆ linear, cyclic and branched hydrocarbylenes, which are optionally substituted and/or interrupted with one or more heteroatoms, and combinations thereof; and
wherein the azanaphthalene moiety is optionally substituted with one or more substituents, preferably selected from the group consisting of halides, alkyls and alkoxides, optionally substituted with one or more heteroatoms.

9. Nanoparticle according to any of claim 7-8, wherein Z¹ and optionally Z² independently represent a moiety of formula (I_{Z2}), preferably a moiety of formula (I_{Z3}), 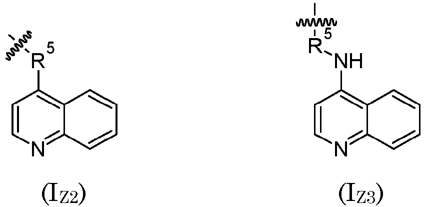
wherein R⁵ is selected from the group consisting of C₁-C₄₀, preferably C₁-C₁₀, more preferably C₁-C₆ linear, cyclic and branched hydrocarbylenes, which are optionally substituted and/or interrupted with one or more heteroatoms, and combinations thereof; and
wherein the azanaphthalene moiety is optionally substituted with one or more substituents, preferably selected from the group consisting of halides, alkyls and alkoxides, optionally substituted with one or more heteroatoms.

10. Nanoparticle according to any of claims 7-9, wherein Z¹ and optionally Z² independently represent a moiety of formula (I_{Z4}) 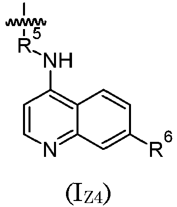
wherein R⁵ is as defined in claim 9, preferably wherein R⁵ represents a C₁-C₁₀ alkylene, more preferably a C₁-C₆ alkylene, most preferably a C₁-C₄ alkylene, and combinations thereof; and
wherein R⁶ is selected from the group consisting of halides, alkoxides alkyls and mixtures thereof, optionally substituted with one or more heteroatoms, preferably halides, more preferably wherein R⁶ is Cl.

11. Nanoparticle according to any of the previous claims, wherein said polyamine segment has a structure according to any of formulae (IIa), (IIb), (IIc) and (IId), and combinations thereof, 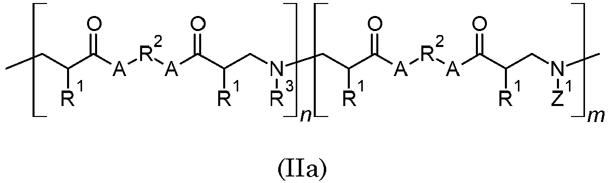 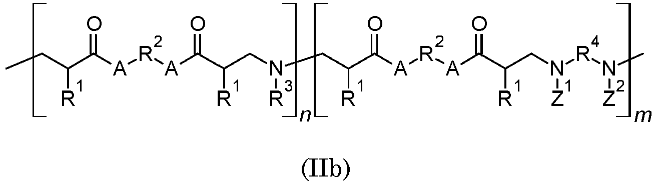 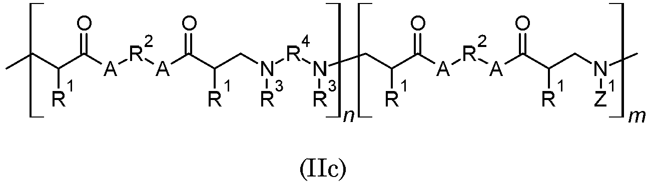 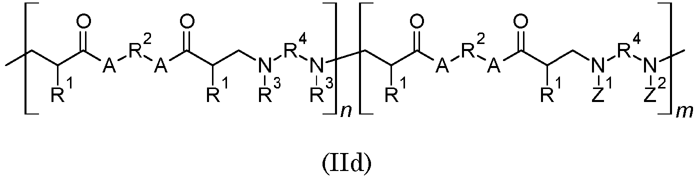 wherein *n, m,* A, R¹, R², R³, R⁴, Z¹ and Z² are each individually as defined in any of the previous claims.

12. Nanoparticle according to any of the previous claims, wherein said polyamine segment, preferably said polymer, has a structure according to formula (III),
{[X-B]ₙ-[X-Q]ₘ}_{q}-T (III)
preferably according to any of formulae (IVa), (IVb), (IVc), (IVd), and combinations thereof, 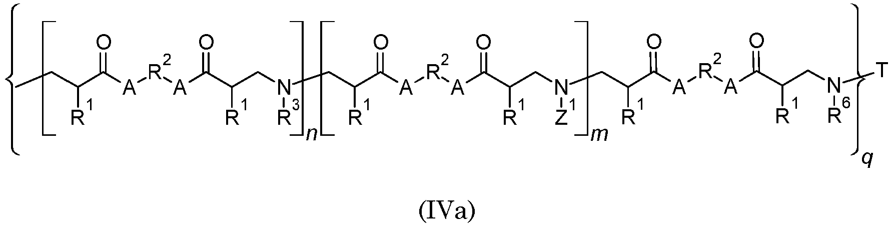 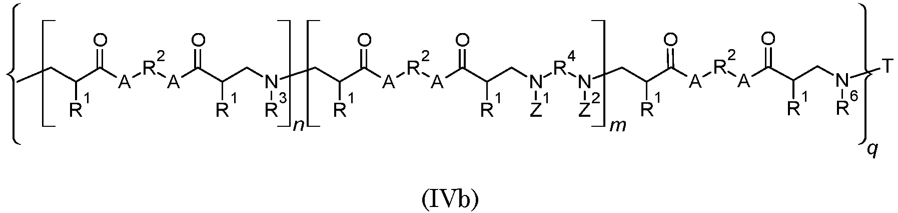 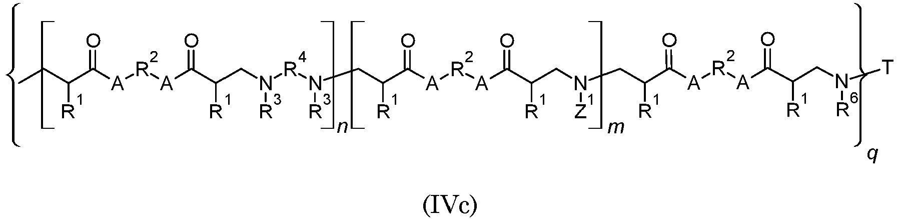 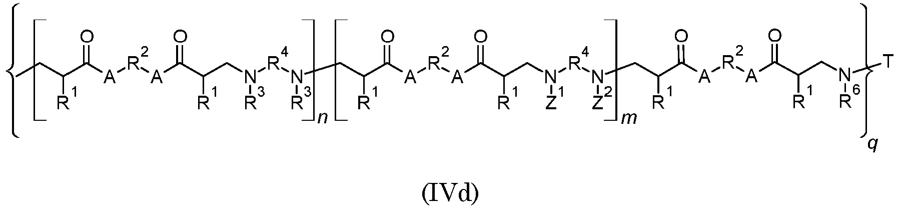
wherein *n, m,* A, R¹, R², R³, R⁴, Z¹ and Z² are each individually as defined in any of the previous claims;
wherein T represents a core having a weight average molecular weight M_{w} of about 60 to about 25000;
*q* is in the range of 1 to 64; and
R⁶ is selected from the group consisting of H, C₁-C₄₀ linear, cyclic and branched hydrocarbyls, which are optionally substituted and/or interrupted with one or more heteroatoms, and combinations thereof.

13. Nanoparticle according to any of the previous claims, wherein the ratio *m* to *n* is in the range of 1:20 or more, preferably in the range of 1:10 to 10:1, more preferably in the range of 1:5 to 2:1, most preferably in the range of 1:4 to 1:1 such as about 1:3.

14. Nanoparticle according to any of the previous claims, wherein the biologically active component comprises an RNA, DNA or derivatives thereof.

15. Nanoparticles in accordance with any of the previous claims for use in a medical method, preferably for use in a medical method as a vaccine.

16. Use of the nanoparticle in accordance with any of the previous claims 1-14 in *in vitro* transfection with nucleic acids in eukaryotic cells.

## Patentansprüche

1. Nanopartikel, umfassend ein Azanaphthalin-funktionalisiertes Polymer und eine biologisch aktive Komponente, ausgewählt aus der Gruppe, bestehend aus Nukleotiden, Polynukleotiden, Oligonukleotiden, Peptiden, Proteinen und Kombinationen davon, wobei das Polymer ein Polyaminsegment der Formel (I) umfasst,
-[X-B]ₙ-[X-Q]ₘ- (I)
wobei X auf einem bis(α,β-ungesättigten Carbonyl)-Monomer basiert, B auf einem Aminmonomer basiert, Q auf einem Azanaphthalin-haltigen Aminmonomer basiert, umfassend eine Azanaphthalineinheit, *m* mindestens 1 ist, und *n* mindestens 1 ist, vorzugsweise *n* + *m* im Bereich von 2-250 ist.

2. Nanopartikel nach Anspruch 1, wobei die Azanaphthalineinheit ausgewählt ist aus der Gruppe, bestehend aus Benzopyridinen einschließlich 1-Benzazinen (Chinolinen) und 2-Benzazinen (Isochinolinen) und Diazanaphthalinen einschließlich 1,2-Diazanaphthaline (Cinnoline), 1,3-Diazanaphthaline (Chinazoline), 1,4-Diazanaphthaline (Chinoxaline), 2,3-Diazanaphthaline (Phthalazine), 1,8-Diazanaphthaline, 1,7-Diazanaphthaline, 2,7-Diazanaphthaline, 1,6-Diazanaphthaline, 1,5-Diazanaphthaline und 2,6-Diazanaphthaline, vorzugsweise Benzopyridine, bevorzugter Chinoline.

3. Nanopartikel nach einem der vorstehenden Ansprüche, wobei X auf einem Bis(α,β-ungesättigten Carbonyl)-Monomer nach Formel (Iₓ) basiert, 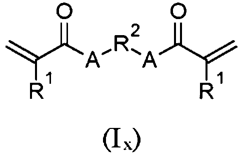 wobei
A ausgewählt ist aus der Gruppe, bestehend aus C(R¹)₂, O, N(R¹), S und Kombinationen davon,
R¹ ausgewählt ist aus der Gruppe, bestehend aus H, Halogeniden und C₁₋C₂₂-Kohlenwasserstoffen wie Alkylen, Alkenylen und Polyalkenylen, die optional mit einem oder mehreren Halogeniden substituiert sind, und Kombinationen davon; und
R² ausgewählt ist aus der Gruppe, bestehend aus linearen, zyklischen und verzweigten C₁₋C₄₀-Kohlenwasserstoffen, die optional substituiert und/oder durch ein oder mehrere Heteroatome unterbrochen sind, und Kombinationen davon.

4. Nanopartikel nach einem der Ansprüche 1 bis 2, wobei X eine Disulfideinheit umfasst, wobei X vorzugsweise wie in Anspruch 3 definiert ist und wobei R² eine Disulfideinheit umfasst, wobei R² vorzugsweise ausgewählt ist aus der Gruppe, bestehend aus C₁-C₁₀-Kohlenwasserstoffen, vorzugsweise linearen Alkylenen, unterbrochen durch mindestens eine Disulfideinheit, und Kombinationen davon.

5. Nanopartikel nach einem der vorstehenden Ansprüche, wobei B auf einem primären Amin nach Formel (I_{B1}) basiert, 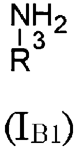
oder auf einem Diamin nach Formel (I_{B2}), 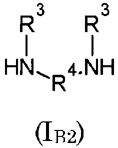
wobei
R³ unabhängig ausgewählt ist aus der Gruppe, bestehend aus H, linearen, zyklischen und verzweigten C₁- C₄₀-Kohlenwasserstoffen, die optional substituiert und/oder mit einem oder mehreren Heteroatomen unterbrochen sind, und Kombinationen davon; und R4 ausgewählt ist aus der Gruppe, bestehend aus linearen, zyklischen und verzweigten C₁-C₄₀-Kohlenwasserstoffen, die optional substituiert und/oder mit einem oder mehreren Heteroatomen unterbrochen sind, und Kombinationen davon.

6. Nanopartikel nach einem der Ansprüche 1 bis 4, wobei B eine Hydroxyleinheit umfasst, vorzugsweise wobei B wie in Anspruch 5 definiert ist und wobei R³ eine Hydroxyeinheit umfasst, bevorzugter, wobei R³ ausgewählt ist aus der Gruppe, bestehend aus C₁-C₁₀-Kohlenwasserstoffhydroxid, vorzugsweise linearem C₁-C₁₀-Alkylhydroxid, und Kombinationen davon.

7. Nanopartikel nach einem der vorstehenden Ansprüche, wobei Q auf einem primären Amin nach Formel (I_{Q1}) basiert, 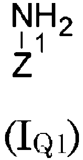
und/oder auf einem Diamin nach der Formel (I_{Q2}), 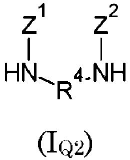
wobei
Z¹ eine Gruppe darstellt, umfassend die Azanaphthalineinheit;
Z² eine Gruppe darstellt, umfassend die Azanaphthalineinheit oder ausgewählt ist aus der Gruppe, bestehend aus H, linearen, cyclischen und verzweigten C₁-C₄₀-Kohlenwasserstoffen, die optional substituiert und/oder mit einem oder mehreren Heteroatomen unterbrochen sind, und Kombinationen davon;
R⁴ wie in Anspruch 5 definiert ist.

8. Nanopartikel nach dem vorstehenden Anspruch, wobei Z¹ und optional Z² unabhängig voneinander eine Einheit nach einer der folgenden Strukturen darstellen, 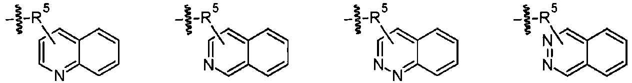 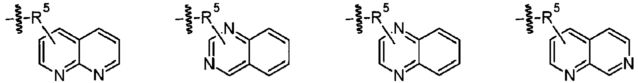 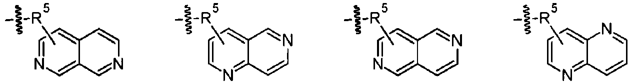
wobei R⁵ ausgewählt ist aus der Gruppe, bestehend aus linearen, cyclischen und verzweigten C₁-C₄₀-, vorzugsweise C₁-C₁₀-, bevorzugter C₁-C₆-Kohlenwasserstoffen, die optional substituiert und/oder mit einem oder mehreren Heteroatomen unterbrochen sind, und Kombinationen davon; und
wobei die Azanaphthalineinheit optional mit einem oder mehreren Substituenten substituiert ist, vorzugsweise ausgewählt aus der Gruppe, bestehend aus Halogeniden, Alkylen und Alkoxiden, optional substituiert mit einem oder mehreren Heteroatomen.

9. Nanopartikel nach einem der Ansprüche 7 bis 8, wobei Z¹ und optional Z² unabhängig voneinander eine Einheit der Formel (I_{Z2}), vorzugsweise eine Einheit der Formel (I_{Z3}), darstellen, 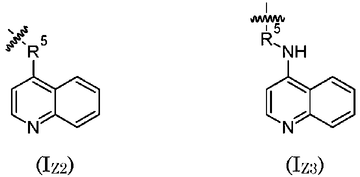
wobei R⁵ ausgewählt ist aus der Gruppe, bestehend aus linearen, cyclischen und verzweigten C₁-C₄₀-, vorzugsweise C₁-C₁₀-, bevorzugter C₁-C₆-Kohlenwasserstoffen, die optional substituiert und/oder mit einem oder mehreren Heteroatomen unterbrochen sind, und Kombinationen davon; und
wobei die Azanaphthalineinheit optional mit einem oder mehreren Substituenten substituiert ist, vorzugsweise ausgewählt aus der Gruppe, bestehend aus Halogeniden, Alkylen und Alkoxiden, optional substituiert mit einem oder mehreren Heteroatomen.

10. Nanopartikel nach einem der Ansprüche 7 bis 9, wobei Z¹ und optional Z² unabhängig voneinander eine Einheit der Formel (I_{Z4}) darstellen, 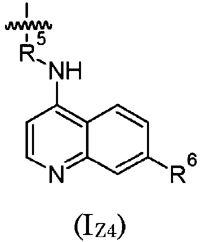
wobei R⁵ wie in Anspruch 9 definiert ist, vorzugsweise wobei R⁵ ein C₁-C₁₀-Alkylen, noch bevorzugter ein C₁-C₆-Alkylen, am meisten bevorzugt ein C₁-C₄-Alkylen und Kombinationen davon darstellt; und
wobei R⁶ ausgewählt ist aus der Gruppe, bestehend aus Halogeniden, Alkoxiden, Alkylen und Mischungen davon, optional substituiert mit einem oder mehreren Heteroatomen, vorzugsweise Halogeniden, bevorzugter wobei R⁶ Cl ist.

11. Nanopartikel nach einem der vorstehenden Ansprüche, wobei das Polyaminsegment eine Struktur nach einer der Formeln (IIa), (IIb), (IIc) und (IId) und Kombinationen davon hat, 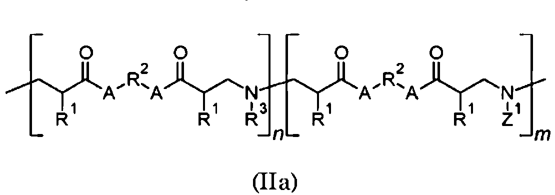 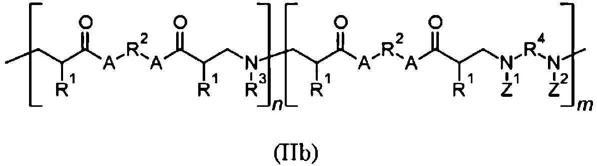 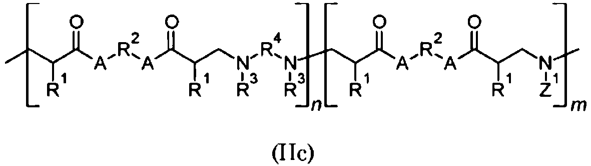 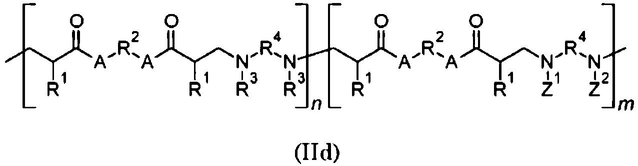 wobei *n, m,* A, R¹, R², R³, R⁴, Z¹ und Z² jeweils individuell wie in einem der vorstehenden Ansprüche definiert sind.

12. Nanopartikel nach einem der vorstehenden Ansprüche, wobei das Polyaminsegment, vorzugsweise das Polymer, eine Struktur nach Formel (III) hat,
{[X-B]ₙ-[X-Q]ₘ}_{q}-T (III)
vorzugsweise nach irgendeiner der Formeln (IVa), (IVb), (IVc), (IVd) und deren Kombinationen, 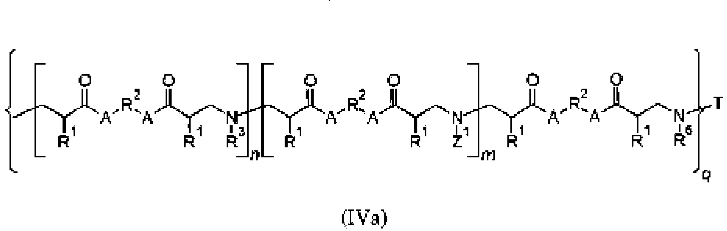 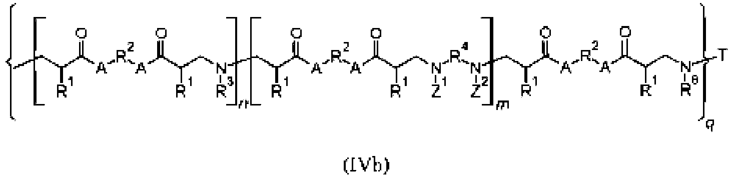 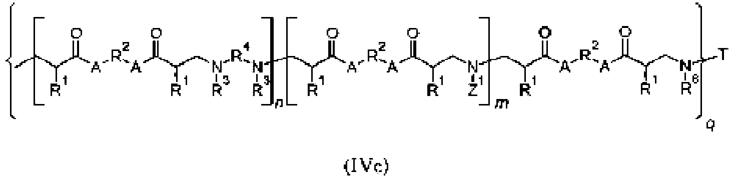 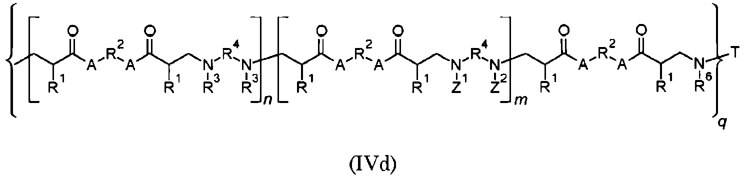
wobei *n, m,* A, R¹, R², R³, R⁴, Z¹ und Z² jeweils individuell wie in einem der vorstehenden Ansprüche definiert sind;
wobei T einen Kern darstellt, mit einem gewichtsmittleren Molekulargewicht M_{w} von etwa 60 bis etwa 25000;
*q* im Bereich von 1 bis 64 ist; und
R⁶ ausgewählt ist aus der Gruppe, bestehend aus H, linearen, cyclischen und verzweigten C₁-C₄₀-Kohlenwasserstoffen, die optional substituiert und/oder mit einem oder mehreren Heteroatomen unterbrochen sind, und Kombinationen davon.

13. Nanopartikel nach einem der vorstehenden Ansprüche, wobei das Verhältnis *m* zu *n* im Bereich von 1:20 oder mehr liegt, vorzugsweise im Bereich von 1:10 bis 10:1, bevorzugter im Bereich von 1:5 bis 2:1, am meisten bevorzugt im Bereich von 1:4 bis 1:1, wie etwa 1:3.

14. Nanopartikel nach einem der vorstehenden Ansprüche, wobei die biologisch aktive Komponente eine RNA, DNA oder Derivate davon umfasst.

15. Nanopartikel nach einem der vorstehenden Ansprüche zur Verwendung in einem medizinischen Verfahren, vorzugsweise zur Verwendung in einem medizinischen Verfahren als Impfstoff.

16. Verwendung des Nanopartikels nach einem der vorstehenden Ansprüche 1 bis 14 bei der *in vitro* Transfektion mit Nukleinsäuren in eukaryotische Zellen.

## Revendications

1. Nanoparticules comprenant un polymère à fonctionnalité azanaphtalène et un composant biologiquement actif choisi dans le groupe constitué par les nucléotides, les polynucléotides, les oligonucléotides, les peptides, les protéines et leurs combinaisons, dans lesquelles ledit polymère comprend un segment polyamine de formule (I), 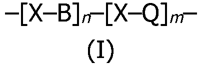 dans laquelle X est basé sur un monomère de bis(carbonyle α,β-insaturé), B est basé sur un monomère d'amine, Q est basé sur un monomère d'amine contenant de l'azanaphtalène comprenant un fragment azanaphtalène, *m* vaut au moins 1, et *n* vaut au moins 1, de préférence la somme *n* + *m* est située dans la plage allant de 2 à 250.

2. Nanoparticule selon la revendication 1, dans laquelle le fragment azanaphtalène est choisi dans le groupe constitué par les benzopyridines incluant les 1-benzazines (quinolines) et les 2-benzazines (isoquinolines) et les diazanaphtalènes incluant les 1,2-diazanaphtalènes (cinnolines), les 1,3-diazanaphtalènes (quinazolines), les 1,4-diazanaphtalènes (quinoxalines), les 2,3-diazanaphtalènes (phtalazines), les 1,8-diazanaphtalènes, les 1,7-diazanaphtalènes, les 2,7-diazanaphtalènes, les 1,6-diazanaphtalènes, les 1,5-diazanaphtalènes, et les 2,6-diazanaphtalènes, de préférence les benzopyridines, mieux encore les quinolines.

3. Nanoparticule selon l'une quelconque des revendications précédentes, dans laquelle X est basé sur un monomère de bis(carbonyle α,β-insaturé) de formule (Iₓ), 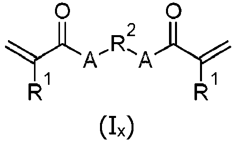 dans laquelle
A est choisi dans le groupe constitué par C(R¹)₂, O, N(R¹), S, et leurs combinaisons,
R¹ est choisi dans le groupe constitué par H, les halogénures et les hydrocarbyles en C₁ à C₂₂ tels que les alkyles, alcényles et polyalcényles, qui sont éventuellement substitués par un ou plusieurs halogénures, et leurs combinaisons ; et
R² est choisi dans le groupe constitué par les hydrocarbylènes linéaires, cycliques et ramifiés en C₁ à C₄₀, qui sont éventuellement substitués et/ou interrompus par un ou plusieurs hétéroatomes, et leurs combinaisons.

4. Nanoparticule selon l'une quelconque des revendications 1 et 2, dans laquelle X comprend un fragment disulfure, de préférence dans laquelle X est tel que défini dans la revendication 3 et dans laquelle R² comprend un fragment disulfure, de préférence dans laquelle R² est choisi dans le groupe constitué par les hydrocarbylènes en C₁ à C₁₀, de préférence les alkylènes linéaires, interrompus par au moins un fragment disulfure, et leurs combinaisons.

5. Nanoparticule selon l'une quelconque des revendications précédentes, dans laquelle B est basé sur une amine primaire de formule (I_{B1}), 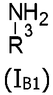 ou sur une diamine de formule (I_{B2}), 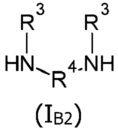 dans laquelle
R³ est indépendamment choisi dans le groupe constitué par H, les hydrocarbyles linéaires, cycliques et ramifiés en C₁ à C₄₀, qui sont éventuellement substitués et/ou interrompus par un ou plusieurs hétéroatomes, et leurs combinaisons ; et
R⁴ est choisi dans le groupe constitué par les hydrocarbylènes linéaires, cycliques et ramifiés en C₁ à C₄₀, qui sont éventuellement substitués et/ou interrompus par un ou plusieurs hétéroatomes, et leurs combinaisons.

6. Nanoparticule selon l'une quelconque des revendications 1 à 4, dans laquelle B comprend un fragment hydroxyle, de préférence dans laquelle B est tel que défini dans la revendication 5 et dans laquelle R³ comprend un fragment hydroxy, mieux encore dans laquelle R³ est choisi dans le groupe constitué par les hydroxy-hydrocarbyles en C₁ à C₁₀, de préférence les hydroxy-alkyles en C₁ à C₁₀ linéaires, et leurs combinaisons.

7. Nanoparticule selon l'une quelconque des revendications précédentes, dans laquelle Q est basé sur une amine primaire de formule (I_{Q1}), 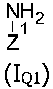 et/ou sur une diamine de formule (I_{Q2}), 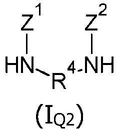 dans laquelle
Z¹ représente un groupe comprenant le fragment azanaphtalène ;
Z² représente un groupe comprenant le fragment azanaphtalène ou est choisi dans le groupe constitué par H, les hydrocarbyles linéaires, cycliques et ramifiés en C₁ à C₄₀, qui sont éventuellement substitués et/ou interrompus par un ou plusieurs hétéroatomes, et leurs combinaisons ;
R⁴ est tel que défini dans la revendication 5.

8. Nanoparticule selon la revendication précédente, dans laquelle Z¹ et éventuellement Z² représentent indépendamment un fragment de l'une quelconque des structures suivantes, 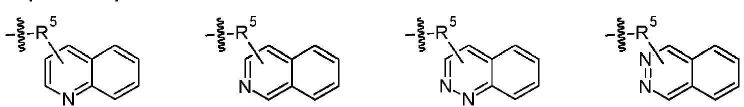 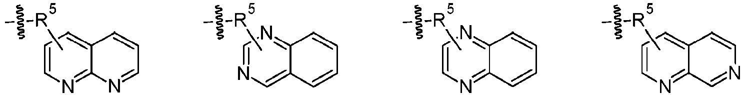 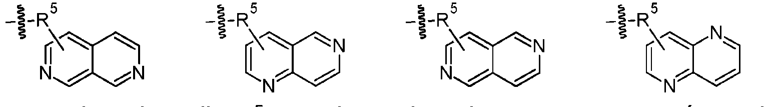
dans laquelle R⁵ est choisi dans le groupe constitué par les hydrocarbylènes linéaires, cycliques et ramifiés en C₁ à C₄₀, de préférence en C₁ à C₁₀, mieux encore en C₁ à C₆, qui sont éventuellement substitués et/ou interrompus par un ou plusieurs hétéroatomes, et leurs combinaisons ; et
dans laquelle le fragment azanaphtalène est éventuellement substitué par un ou plusieurs substituants, de préférence choisis dans le groupe constitué par les halogénures, les alkyles et les alkylates, éventuellement substitués par un ou plusieurs hétéroatomes.

9. Nanoparticule selon l'une quelconque des revendications 7 et 8, dans laquelle Z¹ et éventuellement Z² représentent indépendamment un fragment de formule (I_{Z2}), de préférence un fragment de formule (I_{Z3}), 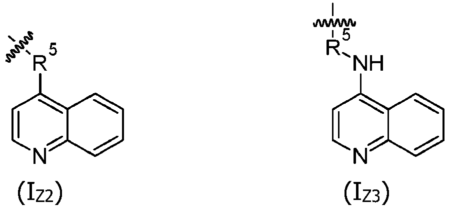
dans laquelle R⁵ est choisi dans le groupe constitué par les hydrocarbylènes linéaires, cycliques et ramifiés en C₁ à C₄₀, de préférence en C₁ à C₁₀, mieux encore en C₁ à C₆, qui sont éventuellement substitués et/ou interrompus par un ou plusieurs hétéroatomes, et leurs combinaisons ; et
dans laquelle le fragment azanaphtalène est éventuellement substitué par un ou plusieurs substituants, de préférence choisis dans le groupe constitué par les halogénures, les alkyles et les alkylates, éventuellement substitués par un ou plusieurs hétéroatomes.

10. Nanoparticule selon l'une quelconque des revendications 7 à 9, dans laquelle Z¹ et éventuellement Z² représentent indépendamment un fragment de formule (I_{Z4}) 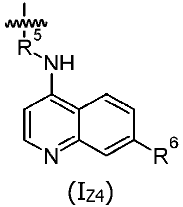
dans laquelle R⁵ est tel que défini dans la revendication 9, de préférence dans laquelle R⁵ représente un alkylène en C₁ à C₁₀, mieux encore un alkylène en C₁ à C₆, tout spécialement un alkylène en C₁ à C₄, et leurs combinaisons ; et
dans laquelle R⁶ est choisi dans le groupe constitué par les halogénures, les alkylates, les alkyles et leurs mélanges, éventuellement substitués par un ou plusieurs hétéroatomes, de préférence un ou plusieurs halogénures, mieux encore dans laquelle R⁶ est Cl.

11. Nanoparticule selon l'une quelconque des revendications précédentes, dans laquelle ledit segment polyamine a une structure de l'une quelconque des formules (IIa), (IIb), (IIc) et (IId), et leurs combinaisons, 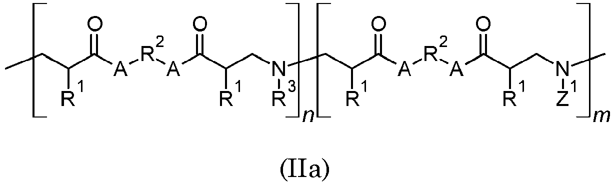 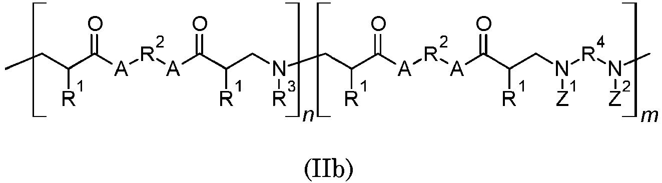 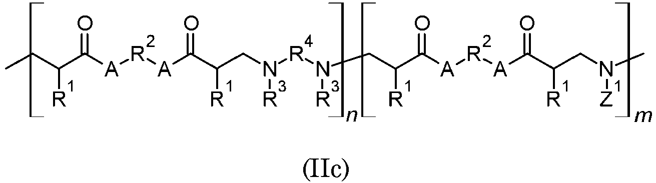 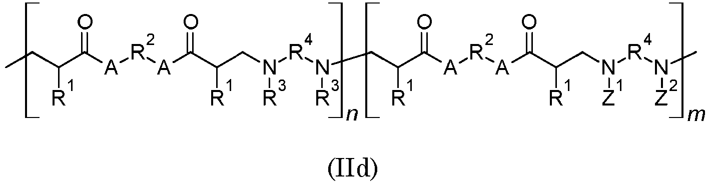 dans laquelle chacun de *n, m*, A, R¹, R², R³, R⁴, Z¹ et Z² est individuellement tel que défini dans l'une quelconque des revendications précédentes.

12. Nanoparticule selon l'une quelconque des revendications précédentes, dans laquelle ledit segment polyamine, de préférence ledit polymère, a une structure de formule (III), 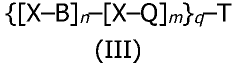
de préférence de l'une quelconque des formules (IVa), (IVb), (IVc), (IVd) et leurs combinaisons, 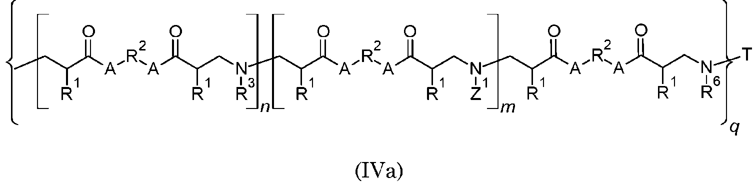 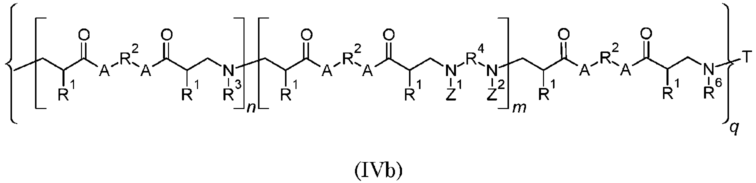 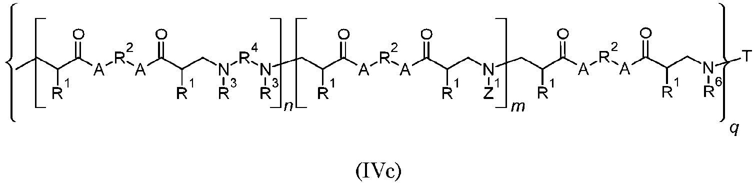 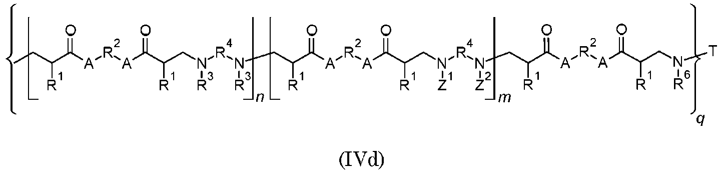
dans laquelle chacun de *n, m*, A, R¹, R², R³, R⁴, Z¹ et Z² est individuellement tel que défini dans l'une quelconque des revendications précédentes ;
dans laquelle T représente un noyau ayant une masse moléculaire moyenne en masse M_{w} d'environ 60 à environ 25 000 ;
*q* est situé dans la plage allant de 1 à 64 ; et
R⁶ est choisi dans le groupe constitué par H**,** les hydrocarbyles linéaires, cycliques et ramifiés en C₁ à C₄₀, qui sont éventuellement substitués et/ou interrompus par un ou plusieurs hétéroatomes, et leurs combinaisons.

13. Nanoparticule selon l'une quelconque des revendications précédentes, dans laquelle le rapport de *m* à *n* est situé dans la plage de 1/20 ou plus, de préférence dans la plage allant de 1/10 à 10/1, mieux encore dans la plage allant de 1/5 à 2/1, tout spécialement dans la plage allant de 1/4 à 1/1, tel qu'environ 1/3.

14. Nanoparticule selon l'une quelconque des revendications précédentes, dans laquelle le composant biologiquement actif comprend un ARN, un ADN, ou des dérivés de ceux-ci.

15. Nanoparticule selon l'une quelconque des revendications précédentes, pour une utilisation dans un procédé médical, de préférence pour une utilisation dans un procédé médical en tant que vaccin.

16. Utilisation de la nanoparticule de l'une quelconque des revendications 1 à 14 dans une transfection *in vitro* avec des acides nucléiques dans des cellules eucaryotes.
